# EUROPEAN PATENT APPLICATION

(11) **EP 4 212 102 A1**
(43) Date of publication of application: **19.07.2023**
(21) Application number: 22151811.1
(22) Date of filing: 17.01.2022
(51) Int. Cl.: A61B 6/03, A61B 6/00, A61G 3/00

(54) **MEDICAL SCANNING SYSTEM, MEDICAL VEHICLE AND METHOD FOR OPERATING A MEDICAL SCANNING SYSTEM**

(71) Applicant: Koninklijke Philips N.V., 5656 AG Eindhoven (NL)
(72) Inventor: JOHNSON, Mark Thomas, Eindhoven (NL); WEISS, Steffen, Eindhoven (NL); BUSSA, Nagaraju, Eindhoven (NL); VOGTMEIER, Gereon, Eindhoven (NL)
(74) Representative: Philips Intellectual Property & Standards

(57) **Abstract**

The invention relates to a medical scanning system (2) for use in a moving vehicle (1). The medical scanning system (2) comprises a rotating component (3) that is configured to rotate around a rotation axis (R) and means for limiting gyroscopic forces caused by the rotating component (3) when the rotation axis (R) changes its direction. The invention further relates to a medical vehicle (1) comprising a medical scanning system (2) according to the above description. The intention also relates to a method for operating a medical scanning system (2) in a moving vehicle (1).

## Description

### FIELD OF THE INVENTION

The invention relates to a medical scanning system for use in a moving vehicle, to a vehicle comprising said medical scanning system and to a method for operating a medical scanning system in a moving vehicle.

### BACKGROUND OF THE INVENTION

Medical scanning systems may be included in vehicles. During transportation, the medical scanning system can be used for some medical scans and in some cases, like a mobile clinic in a ship, performing medical scans during transportation is the common usage of the medical scanning system. This will subject the medical scanning system to forces caused by the motion of the vehicle. This is particularly the case for medical scanning systems such as computed tomography (CT) systems where there are rotating components such as the gantry which weighs about a ton and rotates at up to more than 4Hz. Another example are the rotating anodes of X-ray tubes of standard X-ray systems (C-arm systems, tomosynthesis systems, dental systems).

While the associated forces are constant in nature during normal operation in a stationary setting and as such are accounted for in the construction of the medical scanning system, additional non-constant forces are induced on the rotating parts, if the rotating part is in motion, as is the case when the CT or X-ray system is scanning whilst the vehicle is moving. Said non-constant forces are accelerative, centrifugal and especially also gyroscopic forces. All of these but especially the latter can induce excessive strains on the support of the rotating part and may cause damage to the medical scanning system, require a recalibration of the system and/or have an adverse influence on the image quality, which are generally not encountered if the system is stationary.

### SUMMARY OF THE INVENTION

It is an object of the present invention to provide a medical scanning system for use in a vehicle that can effectively scan while the vehicle is in motion. It is a further object of the present invention to provide a medical vehicle with said medical scanning system and a method for operating a medical scanning system in a moving vehicle that provides effective scans while the vehicle is in motion.

In an aspect of the present invention, a medical scanning system for use in a moving vehicle is provided.

The moving vehicle has a vertical axis, a longitudinal axis and a transverse axis. These axes refer to the vehicle in normal orientation and move together with the vehicle when the vehicle turns and/or tilts. The longitudinal axis extends in a direction of a straight motion of the vehicle and the transverse axis is perpendicular to both the vertical axis and the longitudinal axis.

The medical scanning system comprises a rotating component that is configured to rotate around a rotation axis. It further comprises a bearing that supports said rotating component and many other components that depend on the exact type of medical scanning system.

The medical scanning system further comprises means for limiting gyroscopic forces caused by the rotating component when the rotation axis changes its direction. These gyroscopic forces depend on the mass and the rate of rotation of the rotating part as well as on the rate and orientation at which the rotation axis changes its direction.

The means for limiting gyroscopic forces enable medical scans while the vehicle is in motion such that the bearings supporting the rotating part and other components are not under undue stress. In many cases, performing said medical scans in the moving vehicle can save precious time for a patient and/or might be the only option to get short term diagnostic information.

According to an embodiment, the medical scanning system is a Computed Tomography (CT) system. In this case, the rotating component is in a particular a CT gantry. Alternatively or additionally, the medical scanning system is an X-ray system. In this case, the rotating component is in particular an X-ray anode. While the CT system and the X-ray system are very important examples of medical scanning systems that comprise rotating components, the invention also applies to other medical scanning systems, medical monitoring systems and/or medical treatment systems with rotating components.

According to an embodiment, the vehicle is a truck, a train, a plane, a helicopter, an autonomous flight object and/or a ship. In all of said vehicles, a medical scanning system may be installed and medical scans may be or have to be, for the well-being of a patient, performed while the vehicle is in motion. Safely performing such a medical scan while the vehicle is in motion may be essential for the prognosis of the patient.

According to an embodiment, the means for limiting gyroscopic forces comprise a computing unit and sensors and/or interfaces to sensors. Here, the sensors are sensors specific to the means for limiting gyroscopic forces, while the interfaces to sensors provide access to sensor readings of sensors that are not specific to the means for limiting gyroscopic forces, in particular, to other sensors of the medical scanning system and to sensors of the vehicle.

The computing unit is adapted to receive sensor readings generated by said sensors and to determine and/or predict the gyroscopic forces based on the sensor readings. The determination and/or prediction of the gyroscopic forces allows the medical scanning system or operators of the medical scanning system to take measures to limit said gyroscopic forces.

Examples for the sensors are a camera, a motion sensor, a motor sensor, a compass, a speedometer, a navigation system, a wind sensor, a wave sensor, an altitude sensor, a weather forecast, an acceleration sensor, an inertial measurement unit, a force sensor and a vibration sensor. The navigation system may be a satellite navigation system such as GPS, GLONASS, Galileo or Beidou or may be based on an external application.

The determination of the gyroscopic forces by the computing unit may be a direct determination based on the readings of the motion sensor, the inertial measurement unit, the force sensor or the vibration sensor. The readings of said sensors are directly linked to accelerations and rotations of the medical scanning system. With the knowledge of said accelerations and/or rotations, the known moment of inertia of the rotating component and the known orientation of the rotation axis as well as the known angular speed of the rotating component, the gyroscopic torque can easily be computed.

The prediction of gyroscopic forces depend on the actual vehicle. For example, for a truck, the navigation system provides the current location of the truck and may further provide information about the road ahead of the truck, in particular of curves. Said information on the road ahead of the truck may be additionally or alternatively be determined from an analysis of images of the road ahead of the truck taken by a camera. Further information for the prediction of the gyroscopic forces may be provided, e.g., by speed limits on the road ahead of the truck and/or traffic information. Also, the current reading of the speedometer may enter the prediction of the gyroscopic forces. All or part of this information is used to predict the motion of the truck, in particular rotations of the truck around the vertical axis, which are then used together with the known moment of inertia of the rotating component and the known orientation of the rotation axis as well as the known angular speed of the rotating component to compute the predicted gyroscopic torque.

As another example, for a train, the prediction of the movement of the train is based, inter alia, on information on the rail tracks ahead of the train and current and predicted speed of the train.

For an aerial vehicle such as a plane, a helicopter or an autonomous flight object, a prediction of the movement of the vehicle may be based, inter alia, on the predicted flight route of the aerial vehicle. Further information entering the prediction of the movement that is then used to predict the gyroscopic torque may come from wind sensors and weather forecasts that might predict winds and turbulences.

As another example, for a ship, the prediction of the movement of the ship may be based on the predicted route of the ship. The movement of the ship may also depend on waves which may be measured and/or predicted by a wave sensor as well inferred from an analysis of camera images taken of the waves.

According to an embodiment, the means for limiting gyroscopic forces comprise an orientation of the rotation axis along the vertical axis. With said orientation of the rotation axis, gyroscopic forces that come from a rotation of the vehicle around its vertical axis are reduced or eliminated, since the axis of rotation of the vehicle is then parallel to the axis of rotation of the rotating component.

According to an embodiment, the means for limiting gyroscopic forces comprise an adjustable orientation of the rotation axis. In particular, the orientation of the rotation axis may be adjusted between a rotation axis that is perpendicular to the vertical axis, e.g., a rotation axis along the longitudinal axis or a rotation axis along the transverse axis and a rotation axis that is parallel to the vertical axis. A rotation axis perpendicular to the vertical axis results in the greatest gyroscopic forces when the vehicle turns around its vertical axis, but is in many cases preferable from a medical point of view. On the other hand, with a rotation axis parallel to the vertical axis, the gyroscopic forces are reduced or eliminated, as explained above, but this may be not preferred from a medical point of view. Hence, by adjusting the orientation of the rotation axis, a compromise between gyroscopic forces and medical requirements may be made. In particular, the computing unit may be adapted to control the orientation of the rotation axis based on the determined and/or predicted gyroscopic forces. Hence, based on the predicted motion of the vehicle, different orientations of the rotation axis may be chosen to provide the optimal compromise between gyroscopic forces and medical requirements.

According to an embodiment, the means for limiting gyroscopic forces comprise a modular construction of the rotating component comprising at least two modules and selection means to select which of the modules are rotated during operation of the medical scanning system. By selecting the modules, the mass of the rotating component is adjusted and hence the amount of the gyroscopic force is changed. In particular, given a predicted motion of the vehicle, the module or modules can be chosen that lead to acceptable gyroscopic forces. As an example, the modules may be rings of sources and sensors of a CT gantry. Using all of the modules, a higher resolution and/or shorter scanning time will be achieved while the gyroscopic forces will be maximal. On the hand, using just one of the modules, a lower resolution and/or a longer scanning time will be achieved while the gyroscopic forces are minimized. In particular, the computing unit is adapted to control the selection means based on the determined and/or predicted gyroscopic forces. Hence, based on the predicted motion of the vehicle, the modules used for the medical scan may be chosen by the computing unit.

According to an embodiment, the means for limiting gyroscopic forces comprise passive means to lessen the gyroscopic forces. Said passive means may comprise a bearing allowing motion of the rotating component around an axis perpendicular to an axis around which the medical scanning system rotates, in particular the vertical axis, and the rotation axis, springs, elastic components, and damping components. By allowing a motion of the rotating component around an axis perpendicular to the axis around which the medical scanning system rotates and the rotation axis, a gyroscopic torque will lead to a rotation of the rotating component around said axis. Hence, the gyroscopic torque has no longer to be fully counteracted by the bearing supporting the rotating component, and therefore the gyroscopic forces on the bearing a reduced. In the case of a medical vehicle moving along a curvy path in the horizontal plane, the axis around which the medical scanning system rotates is the vertical axis. For most practical purposes, the rotation around said axis has to be limited, which is in particular performed by the springs, elastic components and/or damping components.

According to an embodiment, the means for limiting gyroscopic forces comprise active means to obviate the gyroscopic forces. Said active means may comprise rotational electric motors, linear electric actuators, gearing components and damping components. Further, the computing unit is configured to control the active means based on the determined and/or predicted gyroscopic forces. Hence, for example, a rotation of the rotating component around an axis perpendicular to the vertical axis and the rotation axis may be forced by the rotational electric motors or by linear electric actuators arranged at either end of the rotating component. Said forced rotation may be such that a part or all of the gyroscopic torque due a change of the direction of the rotation axis is counteracted, hence reducing or eliminating the gyroscopic force on the bearing supporting the rotating component. The amount of motion caused by the active means may be, in particular, controlled by the computing unit such that the gyroscopic forces on the bearings do not exceed a predetermined value, e.g., the maximum specifications of said bearings.

According to an embodiment, the computing unit is configured to issue a speed limit for the vehicle and/or to limit the rotation speed of the rotating component. By issuing a speed limit, given a predicted trajectory of the vehicle, the amount of rotation of the vehicle is limited and hence the gyroscopic forces are limited. Also, by limiting the rotation speed of the rotating component, the gyroscopic forces are limited. In particular, the computing unit may determine the best compromise between limiting the speed of the vehicle and limiting the rotation speed of the rotating component, while keeping the gyroscopic forces below a predetermined limit. Additionally or alternatively, the computing unit is configured to schedule a medical scan based on the determined and/or predicted gyroscopic forces. As an example, if a road ahead of a truck has curvy parts and straight parts, the gyroscopic forces for the straight parts will be smaller than those for the curvy parts of the road and hence the computing unit may schedule a medical scan for the time when the vehicle is on the straight parts of the road.

In another aspect of the present invention, a medical vehicle comprising a medical scanning system according to the above description is provided. In particular, with said medical scanning system, gyroscopic forces caused by the rotating component are limited and hence a medical scan can be performed while the medical vehicle is in motion. Further advantages are given in the above description.

In yet another aspect of the present invention, a method for operating a medical scanning system in a moving vehicle is provided. The medical scanning system comprises a rotating component that is configured to rotate around a rotation axis and means for limiting gyroscopic forces caused by the rotating component when the rotation axis changes its direction. Said means for limiting gyroscopic forces comprise a computing unit and sensors and/or interfaces to sensors. The sensors may comprise a camera, a motion sensor, a motor sensor, a compass, a speedometer, a navigation system, a wind sensor, a wave sensor, an altitude sensor, a weather forecast, an acceleration sensor, an inertial measurement unit, a force sensor and a vibration sensor.

According to the method, sensor readings are generated by the sensors. Said generated sensor readings are then received by the computing unit and based on the sensor readings, the gyroscopic forces are determined and/or predicted. Based on said determined and/or predicted gyroscopic forces, control commands to limit the gyroscopic forces are transmitted by the computing unit. This enables medical scans while the vehicle is in motion and such that the bearings supporting the rotating part and other components are not under undue stress. In many cases, performing said medical scans in the moving vehicle can save precious time for a patient.

According to an embodiment, the means for limiting gyroscopic forces comprise a modular construction of the rotating component comprising at least two modules and selection means to select which of the modules are rotated during operation of the medical scanning system. The control commands transmitted by the computing unit control the selection means in order to limit the gyroscopic forces. Further details are explained in the above description.

According to an embodiment, the means for limiting gyroscopic forces comprise active means to obviate the gyroscopic forces. Said active means may comprise rotational electric motors, linear electric actuators, gearing components and damping components. The control commands control the active means in order to limit the gyroscopic forces. Further details are explained in the above description.

According to an embodiment, the control commands issue a speed limit for the vehicle, limit the rotation speed of the rotating component and/or schedule a medical scan. This will also limit the gyroscopic forces, as explained in detail in the above description.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic side view of an embodiment of a medical vehicle;
Fig. 2 shows a schematic top view of another embodiment of a medical vehicle;
Fig. 3 shows a schematic side view of an embodiment of a medical scanning system;
Fig. 4 shows a schematic side view of another embodiment of a medical scanning system;
Fig. 5 shows a schematic side view of yet another embodiment of a medical scanning system;
Fig. 6 shows a schematic side view of yet another embodiment of a medical scanning system; and
Fig. 7 shows a schematic side view of yet another embodiment of a medical scanning system.

### DETAILED DESCRIPTION OF EMBODIMENTS

Like numbered elements in these figures are either equivalent elements or perform the same function. Elements which have been discussed previously will not necessarily be discussed in later figures if the function is equivalent.

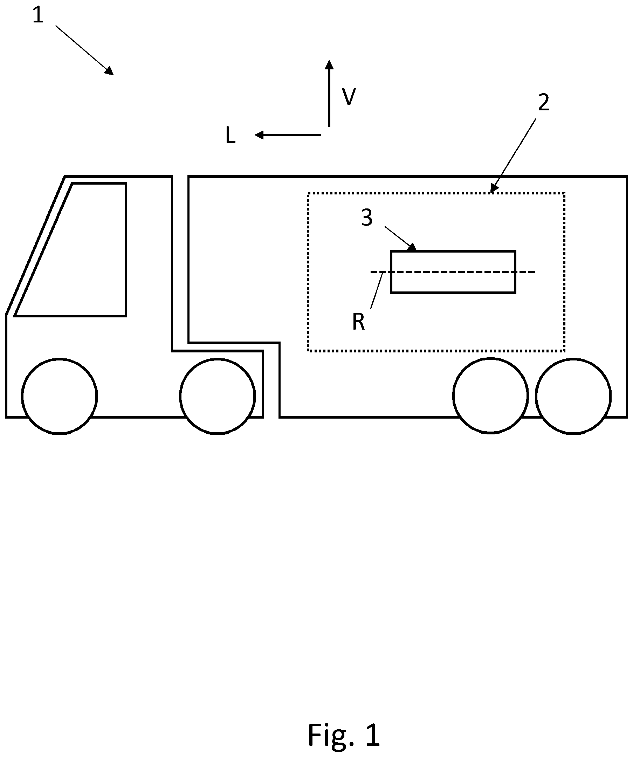

Fig. 1 shows a schematic side view of an embodiment of a medical vehicle 1 with a medical scanning system 2. In this embodiment, the medical vehicle 1 is pictured as a truck, but other medical vehicles such as a train, a plane, a helicopter, an autonomous flight object or a ship may be used equivalently.

The medical vehicle 1 has a vertical axis V and a longitudinal axis L, wherein the longitudinal axis L extends in a direction of a straight motion of the vehicle 1. Further, a transverse axis T is defined as an axis perpendicular to both the vertical axis V and the longitudinal axis L.

The medical scanning system 2 comprises a rotating component 3 that is configured to rotate around a rotation axis R. During a medical scan, i.e., when the rotating component 3 rotates around the rotation axis R, and while the vehicle 1 is in motion, gyroscopic forces may appear due to the rotation of the rotating component 3 when the vehicle 1 rotates around its vertical axis V, i.e., when the vehicle moves along a curve.

In order to limit said gyroscopic forces and hence allow medical scans to be performed while the vehicle 1 is moving, which is desired in many cases and can be of vital importance for a patient, the medical scanning system 2 further comprises means for limiting gyroscopic forces. These means for limiting gyroscopic forces are explained in detail in the following figures.

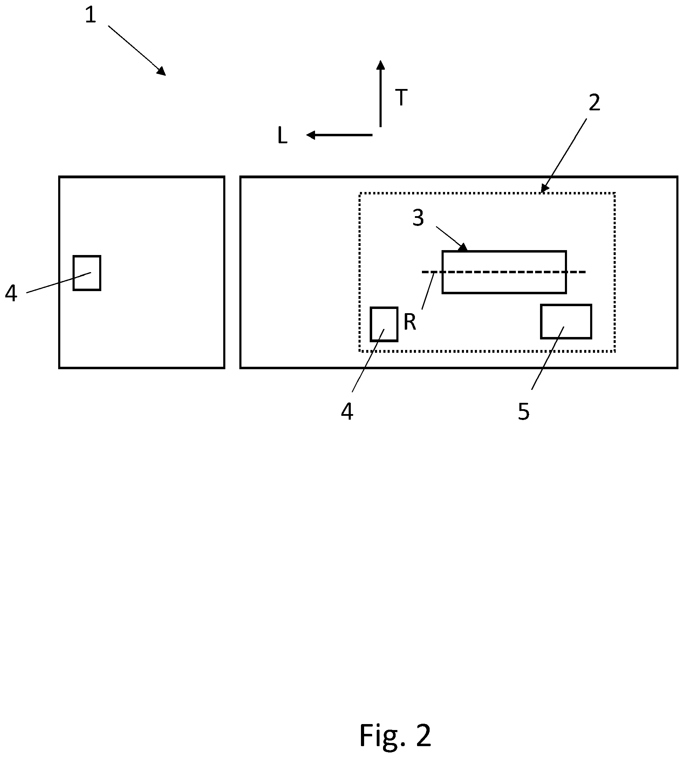

Fig. 2 shows a top view of another embodiment of a medical vehicle 1. The medical vehicle 1 and the medical scanning system 2 comprises sensors 4. If the medical vehicle is a truck, said sensors 4 may comprise a camera, a motion sensor, a motor sensor, a compass, a speedometer, a navigation system, an acceleration sensor, an inertial measurement unit, a force sensor and a vibration sensor. If the medical vehicle is an aerial vehicle, the sensors 4 may further comprise a wind sensor, an altitude sensor and a weather forecast and if the medical vehicle is a ship, the sensors 4 may further comprise a wave sensor. Said sensors are connected (not shown here) via interfaces to a computing unit 5 that can receive the sensor readings generated by the sensors 4. Using said sensor readings, the computing unit 5 determines and/or predicts the motion of the vehicle 1 and from the motion of the vehicle 1 the current or predicted gyroscopic forces. In a truck, for example, the predicted motion may depend on the shape of the road ahead, in particular on curves and straight sections. Further, the motion of the vehicle may depend on the current speed, speed limits and/or traffic ahead. This information is provided, in particular, by the navigation system and can be further supported by the analysis of camera images.

Using the predicted gyroscopic forces, the computing unit 5 may issue a speed limit for the vehicle 1 such that the gyroscopic forces do not exceed a predetermined value during a medical scan. For example, the vehicle 1 may be allowed to run at one speed on straight sections of the road and at another, reduced speed, in a curvy section of the road. Alternatively or additionally, the computing unit 5 may limit the rotation speed of the rotating component 3. Hence, the rotating component 3 may be allowed to rotate at one speed when the vehicle 1 is on a straight section of the road and at another reduced speed when the vehicle 1 is on a curvy section of the road. The reduction of the speed of the vehicle 1 and the rotating speed of the rotating component 3 may be combined to keep the predetermined limit on the gyroscopic forces. Further, given the predicted motion and hence the predicted gyroscopic forces, the computing unit 5 may schedule a medical scan such that the gyroscopic forces are limited. In the example of the truck, a medical scan may be scheduled for a straight section of the road and be paused on curvy sections of the road.

Similar considerations may be made for ships. Here, the prediction of waves and the related movement of the ship have to be processed to determine and predict the motion of the ship. Special sensors of the ship inertial system and the use of wind sensors including the actual navigation information allow for good prediction of the expected ship movements.

Likewise, for the application in a plane, a helicopter or an autonomous flight object, the actual speed, height, inclination as well as information about wind, turbulences and the weather condition have to be taken into account for the prediction of the movement of the vehicle 1.

In the following figures, the computing unit 5 and the sensors 4 are not explicitly shown, however it is understood that they may be present.

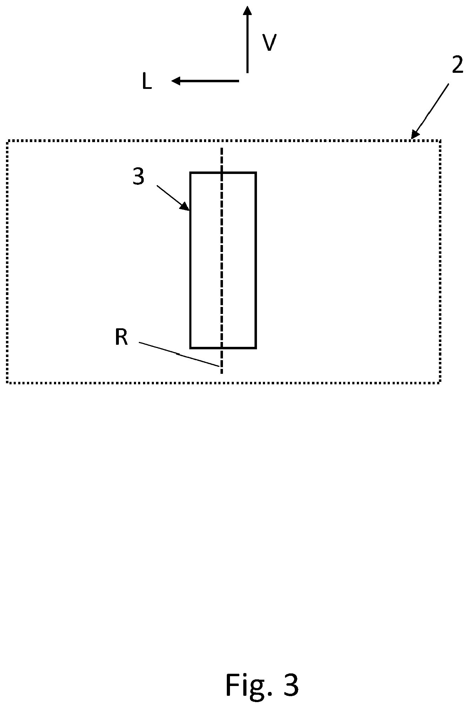

Fig. 3 shows a schematic side view of an embodiment of a medical scanning system 2. In this medical scanning system 2, the rotation axis R of the rotating component 3 is parallel to the vertical axis V. Hence, gyroscopic forces due to a rotation of the vehicle 1 around the vertical axis V are greatly reduced or even eliminated. This configuration, however, can only be used where a placement of the rotation axis R parallel to the vertical axis V is possible from a medical perspective.

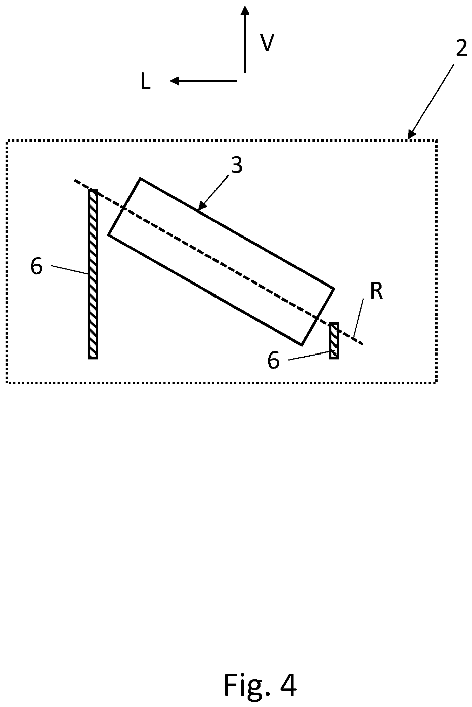

Fig. 4 shows a schematic side view of another embodiment of a medical scanning system 2. In this medical scanning system 2, the rotation axis R of the rotating component 3 is adjustable, in particular from an orientation perpendicular to the vertical axis V, i.e., in a plane spanned by the longitudinal axis and the transverse axis T, to an orientation parallel to the vertical axis V. Said adjustment of the rotation axis R may be made, for example, using a rotation axis adjustment motor 6. The adjustment of the rotation axis R may be made by the computing unit 5 based on the predicted motion of the vehicle 1 and based on medical information. The more the rotation axis R is parallel to the vertical axis V, the smaller the gyroscopic forces. However, depending on, e.g., details of the medical scanning system 2, details of the scan to be performed such as which part of the patient's body is to be scanned, and/or the medical condition of the patient, there may exist limitations to the orientation of the rotation axis R. These limitations will have to be taken into account when the orientation of the rotation axis R is determined by the computing unit 5.

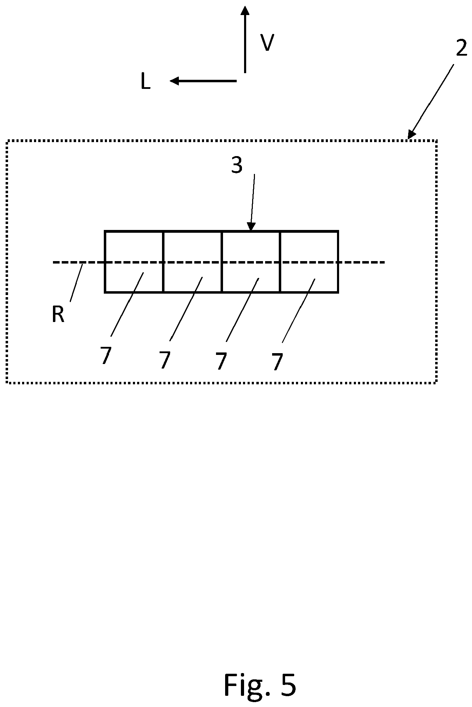

Fig. 5 shows a schematic side view of yet another embodiment of a medical scanning system 2 to be used in a medical vehicle 1 while the vehicle 1 is moving. In this embodiment, the rotating component 3 comprises four modules 7 and selection means (not shown here) to select which of the modules 7 are rotated during the medical scan. Of course, a different number of modules 7 is possible. For a CT system as an example, the modules 7 may be rings comprising sources and sensors. Depending on the predicted motion of the vehicle 1, a certain number of modules 7 may be chosen to perform the medical scan. For example, if a curvy road is ahead, only one module 7 is chosen to perform the medical scan. Hence, only one module is rotated and the gyroscopic force is that of only one module. If, however, a straight road is ahead or a part of the road where the speed of the vehicle 1 is strongly limited, more than one module 7 or even all of the modules 7 may be used to perform the medical scan.

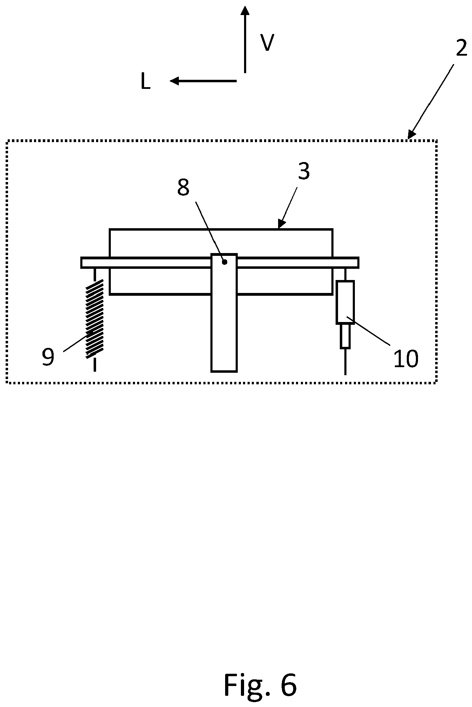

Fig. 6 shows a schematic side view of yet another embodiment of a medical scanning system 2. In this embodiment, a bearing 8 allow the motion of the rotating component 3 around the transverse axis T. In general, a motion of the rotating component 3 around an axis perpendicular to the vertical axis V and the rotation axis R should be allowed. Further, it is possible to allow the rotation around more than one axis. When the rotating component 3 experiences a gyroscopic torque due to a vehicle rotation around the vertical axis, said gyroscopic torque results in a rotation of the rotating component 3 around the transverse axis T and the gyroscopic force exerted on the bearings of the rotating component 3 is reduced or eliminated. In general, said rotation around the transverse axis T can only be allowed to a certain degree, depending, e.g., on details of the medical scanning system 2, details of the scan to be performed such as which part of the patient's body is to be scanned, and/or the medical condition of the patient. In order to limit the rotation around the transverse axis T to said certain degree, springs 9 and damping components 10 are provided.

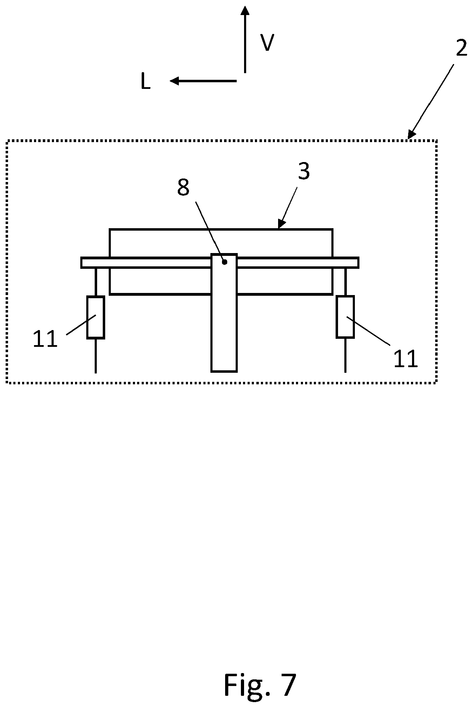

Fig. 7 shows a schematic side view of yet another embodiment of a medical scanning system. Like above, a rotation of the rotating component 3 around the transverse axis T is allowed. However, in this embodiment, said rotation is forced by active means 11, which are pictured as linear electric actuators. Of course, other active means 11 may be used, such as a rotational electric motor. Based on the predicted gyroscopic forces provided by the computing unit 5, the active means perform a rotation of the rotating component 3 around the transverse axis T such that the gyroscopic forces are limited.

In the embodiments of Fig. 4 to Fig. 7, it is to be understood that other elements like the patient table will also be adjusted according to the change in rotation axis R.

While the invention has been illustrated and described in detail in the drawings and foregoing description, such illustration and description are to be considered illustrative or exemplary and not restrictive; the invention is not limited to the disclosed embodiments. In particular, several embodiments may be combined to provide optimal limitation of gyroscopic forces.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: Medical vehicle
- 2: Medical scanning system
- 3: Rotating component
- 4: Sensor
- 5: Computing unit
- 6: Rotation axis adjustment motor
- 7: Module
- 8: Bearing
- 9: Spring
- 10: Damping component
- 11: Active means

- L: Longitudinal axis
- R: Rotation axis
- T: Transverse axis
- V: Vertical axis

## Claims

1. Medical scanning system for use in a moving vehicle (1), wherein the moving vehicle (1) has a vertical axis (V), a longitudinal axis (L) that extends in a direction of a straight motion of the vehicle (1) and a transverse axis (T) that is perpendicular to the vertical axis (V) and to the longitudinal axis (L), the medical scanning system (2) comprising:
a rotating component (3) that is configured to rotate around a rotation axis (R) and
means for limiting gyroscopic forces caused by the rotating component (3) when the rotation axis (R) changes its direction.

2. Medical scanning system according to claim 1, wherein the medical scanning system (2) is a Computed Tomography, CT, system and, in particular, the rotating component (3) is a CT gantry and/or the medical scanning system (2) is an X-ray system and, in particular, the rotating component (3) is an X-ray anode.

3. Medical scanning system according to claim 1 or 2, wherein the vehicle (1) is a truck, a train, a plane, a helicopter, an autonomous flight object and/or a ship.

4. Medical scanning system according to any of claims 1 to 3, wherein the means for limiting gyroscopic forces comprise a computing unit (5) and sensors (4) and/or interfaces to sensors (4), wherein the computing unit (5) is adapted to receive sensor readings generated by the sensors (4) and to determine and/or predict the gyroscopic forces based on the sensor readings and wherein the sensors (4) comprise at least one out of a group consisting of: a camera, a motion sensor, a motor sensor, a compass, a speedometer, a navigation system, a wind sensor, a wave sensor, an altitude sensor, a weather forecast, an acceleration sensor, an inertial measurement unit, a force sensor and a vibration sensor.

5. Medical scanning system according to any of claims 1 to 4, wherein the means for limiting gyroscopic forces comprise an orientation of the rotation axis (R) along the vertical axis (V).

6. Medical scanning system according to any of claims 1 to 5, wherein the means for limiting gyroscopic forces comprise an adjustable orientation of the rotation axis (R) and, in particular, the computing unit (5) is adapted to control the orientation of the rotation axis (R) based on the determined and/or predicted gyroscopic forces.

7. Medical scanning system according to any of claims 1 to 6, wherein the means for limiting gyroscopic forces comprise a modular construction of the rotating component (3) comprising at least two modules (7) and selection means to select which of the modules (7) are rotated during operation of the medical scanning system (2) and, in particular, the computing unit (5) is adapted to control the selection means based on the determined and/or predicted gyroscopic forces.

8. Medical scanning system according to any of claims 1 to 7, wherein the means for limiting gyroscopic forces comprise passive means to lessen the gyroscopic forces and the passive means comprise at least one out of a group consisting of: a bearing (8) allowing motion of the rotating component (3) around an axis perpendicular to an axis around which the medical scanning system (2) rotates, in particular the vertical axis (V), and the rotation axis (R), springs (9), elastic components, and damping components (10).

9. Medical scanning system according to any of claims 4 to 8, wherein the means for limiting gyroscopic forces comprise active means (11) to obviate the gyroscopic forces and the computing unit (5) is configured to control the active means (11) based on the determined and/or predicted gyroscopic forces, wherein the active means (11) comprise at least one out of a group consisting of: rotational electric motors, linear electric actuators, gearing components and damping components.

10. Medical scanning system according to any of claims 4 to 9, wherein the computing unit (5) is configured to issue a speed limit for the vehicle (1), to limit the rotation speed of the rotating component (3) and/or to schedule a medical scan based on the determined and/or predicted gyroscopic forces.

11. Medical vehicle, comprising a medical scanning system (2) according to any of claims 1 to 10.

12. Method for operating a medical scanning system (2) in a moving vehicle (1), the medical scanning system (2) comprising a rotating component (3) that is configured to rotate around a rotation axis (R) and means for limiting gyroscopic forces caused by the rotating component (3) when the rotation axis (R) changes its direction, wherein the means for limiting gyroscopic forces comprise a computing unit (5) and sensors (4) and/or interfaces to sensors (4) and wherein the sensors (4) comprise at least one out of a group consisting of: a camera, a motion sensor, a motor sensor, a compass, a speedometer, a navigation system, a wind sensor, a wave sensor, an altitude sensor, a weather forecast, an acceleration sensor, an inertial measurement unit, a force sensor and a vibration sensor, the method comprising:
generating, by the sensors (4), sensor readings;
receiving, by the computing unit (5), the generated sensor readings;
determining and/or predicting, by the computing unit (5), the gyroscopic forces based on the sensor readings; and
transmitting, by the computing unit (5), control commands to limit the gyroscopic forces based on the determined and/or predicted gyroscopic forces.

13. Method according to claim 12, wherein the means for limiting gyroscopic forces comprise a modular construction of the rotating component (3) comprising at least two modules (7) and selection means to select which of the modules (7) are rotated during operation of the medical scanning system (2) and the control commands control the selection means.

14. Method according to claim 12 or 13, wherein the means for limiting gyroscopic forces comprise active means (11) to obviate the gyroscopic forces and the control commands control the active means (11), and wherein the active means (11)# comprise at least one out of a group consisting of: rotational electric motors, linear electric actuators, gearing components and damping components.

15. Method according to any of claims 12 to 14, wherein the control commands issue a speed limit for the vehicle (1), limit the rotation speed of the rotating component (3) and/or schedule a medical scan.
